(19) Europäisches Patentamt
European Patent Office
Office européen des brevets

(11) Publication number: **0 195 557 B1**

# (12) EUROPEAN PATENT SPECIFICATION

(45) Date of publication of patent specification: **05.10.88**

(21) Application number: **86301572.3**

(22) Date of filing: **06.03.86**

(51) Int. Cl.[4]: **C 07 D 403/04**, C 07 D 401/12, A 61 K 31/41, A 01 N 43/653

## (54) Triazole antifungal agents.

(30) Priority: **14.03.85 GB 8506619**

(43) Date of publication of application: **24.09.86 Bulletin 86/39**

(45) Publication of the grant of the patent: **05.10.88 Bulletin 88/40**

(84) Designated Contracting States: **AT BE CH DE FR GB IT LI LU NL SE**

(56) References cited:
**EP-A-0 096 569**
**EP-A-0 115 400**

**The file contains technical information submitted after the application was filed and not included in this specification**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE CH DE FR IT LI LU NL SE AT**

(72) Inventor: **Richardson, Kenneth, Dr.**
**48, St. Stephens Hill**
**Canterbury Kent (GB)**
Inventor: **Narayanaswami, Subramaniyan, Dr.**
**31, Bruce Close**
**Deal Kent (GB)**

(74) Representative: **Wood, David John et al**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel triazole derivatives which have antifungal activity and are useful in the treatment of fungal infections in animals, including humans, and as agricultural fungicides.

EP—A—96569 and EP—A—115400 describe certain triazole antifungal agents.

According to the invention, there are provided compounds of the formula:—

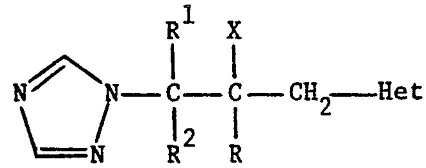

--- (I)

where R is a phenyl group optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group; X is —OH, —F, —Cl or —Br; $R^1$ and $R^2$ are each independently H, $CH_3$ or F; and Het is a 1-tetrazolyl or 2-tetrazolyl group; and the O-esters and O-ethers (as hereinafter defined) of the compounds in which X is —OH; and the pharmaceutically and agriculturally acceptable salts of said compounds and esters and ethers.

$C_3$ and $C_4$ alkyl and alkoxy groups can be straight or branched chain.

The invention also provides a pharmaceutical composition comprising a compound of the formula (I) or an O-ester, O-ether or pharmaceutically acceptable salt thereof, together with a pharmaceutically acceptable diluent or carrier.

The invention further provides a compound of the formula (I) or an O-ester, O-ether or pharmaceutically acceptable salt thereof, for use in medicine, in particular for treating fungal infections in animals, including humans.

The invention yet further provides an antifungal composition for agricultural (including horticultural) use, comprising a compound of the formula (I) or an O-ester, O-ether or agriculturally acceptable salt thereof, together with an agriculturally acceptable diluent or carrier.

The invention yet further provides a method of treating an animal (including a human being), plant or seed having a fungal infection, which comprises treating said animal, plant or seed, or the locus of said plant or seed, with an effective amount of a compound of the formula (I) or O-ester or O-ether thereof or with, as appropriate, a pharmaceutically or agriculturally acceptable salt thereof.

When R is said optionally substituted phenyl group, it is preferably phenyl substituted by 1 to 3 substituents, more preferably 1 or 2 substituents, each independently selected from F, Cl, Br, I and $CF_3$, and most preferably from F and Cl. The preferred individual groups represented by R are 4-fluorophenyl, 4-chlorophenyl, 4-bromophenyl, 4-iodophenyl, 4-trifluoromethylpheny, 2-chlorophenyl, 2,4-dichlorophenyl, 2,4-difluorophenyl, 2-chloro-4-fluorophenyl, 2-fluoro-4-chlorophenyl, 2,5-difluorophenyl, 2,4,6-trifluorophenyl, 4-bromo-2,5-difluorophenyl and 5-chloro-pyrid-2-yl.

X is preferably —OH. $R^1$ and $R^2$ are each preferably H or $CH_3$.

The O-esters are $C_2$—$C_4$ alkanoyl (e.g. acetyl) and benzoyl esters. The phenyl ring of benzoyl esters can be substituted by, for example, 1 or 2 $C_1$—$C_4$ alkyl or halo groups. The O-ethers are $C_1$—$C_4$ alkyl, $C_2$—$C_4$ alkenyl, phenyl-($C_1$—$C_4$ alkyl) and phenyl ethers. Again said phenyl groups can be ring substituted by, e.g., 1 or 2 $C_1$—$C_4$ alkyl or halo groups.

The compounds of the formula (I) in which X is OH can be prepared by reacting an oxirane of the formula:—

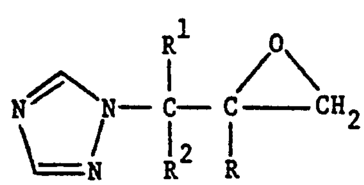

--- (II)

with tetrazole, typically in the presence of bis(tri-*n*-butylstannyl)oxide. The bis(tri-*n*-butylstannyl)oxide is believed to react with tetrazole to form the 1- and 2-tributylstannyl ($Bu_3Sn$—) derivatives thereof which facilitate the ring opening of the epoxide (II). The reaction is typically carried out in a suitable organic solvent, e.g. toluene, at from room temperature up to about 110°C, and desirably under an atmosphere of nitrogen. The reaction produces a mixture of the 1-tetrazolyl and 2-tetrazolyl end products, which can be separated chromatographically.

The oxirane starting materials of the formula (II) are either known compounds or can be prepared by methods analogous to those of the prior art (see e.g. EP 0069442, EP 0044605, US 4,499,281, EP 0122056, EP 0122693, EP 0120276, and EP 0118245).

The compounds of the formula (I) in which X is F, Cl or Br can be prepared by the halogenation of the corresponding compounds in which X is —OH.

The halogenation is carried out according to conventional procedures, e.g. using $SOCl_2$, $SOBr_2$ or

diethylaminosulphur trifluoride ($Et_2NSF_3$), as appropriate (see European patent application publication no. 96569).

In a typical procedure utilizing thionyl chloride or bromide, the hydroxy-containing bis-triazole (II) in a suitable organic solvent, e.g. dry acetonitrile, is reacted with thionyl chloride or bromide at a temperature of from 0°C to reflux temperature, optionally in the presence of a base, e.g. imidazole. The halo-containing product can then be isolated and purified in a conventional manner.

The reaction using diethylaminosulphur trifluoride is typically carried out at a temperature of from about 0°C to room temperature, preferably in methylene chloride as the solvent. Again the product can be isolated and purified conventionally.

The O-esters and O-ethers can be prepared conventionally, typically by reacting an alkali metal salt of compound (I) [X = —OH] with the appropriate chloro- or bromo-compound, e.g. an alkanoyl or benzoyl chloride, or alkyl, alkenyl, benzyl or phenyl chloride or bromide.

Pharmaceutically acceptable acid addition salts of the compounds of the formula (I) are those formed from strong acids which form non-toxic acid addition salts, such as hydrochloric, hydrobromic, sulphuric, oxalic and methanesulphonic acids. Such salts are also useful for agricultural use.

The salts may be obtained by conventional procedures, e.g. by mixing solutions containing approximately equimolar amounts of the free base and desired acid, and the required salt is collected by filtration, if insoluble, or by evaporation of the solvent.

The compounds of the formula (I) and their O-esters, O-ethers and salts are antifungal agents, useful in combating fungal infections in animals, including humans. For example they are useful in treating topical fungal infections in man caused by, among other organisms, species of *Candida, Trichophyton, Microsporum* or *Epidermophyton*, or in mucosal infections caused by *Candida albicans* (e.g. thrush and vaginal candidiasis). They can also be used in the treatment of systemic fungal infections caused by, for example, *Candida albicans, Cryptococcus neoformans, Aspergillus flavus, Aspergillus fumigatus, Coccidioides, Paracoccidioides, Histoplasma* or *Blastomyces.*

The *in vitro* evaluation of the antifungal activity of the compounds can be performed by determining the minimum inhibitory concentration (m.i.c.) which is the concentration of the test compounds, in a suitable medium, at which growth of the particular micro-organism fails to occur. In practice, a series of agar plates, each having the test compound incorporated at a particular concentration is inoculated with a standard culture of, for example, *Candida albicans* and each plate is then incubated for 48 hours at 37°C. The plates are then examined for the presence or absence of growth of the fungus and the appropriate m.i.c. value is noted. Other micro-organisms used in such tests can include *Candida albicans, Aspergillus fumigatus, Trichophyton* spp; *Microsporum* spp; *Epidermophyton floccosum, Coccidioides immitis* and *Torulopsis glabrata.*

The *in vivo* evaluation of the compounds can be carried out at a series of dose levels by intraperitoneal or intravenous injection or by oral administration, to mice which are inoculated with, e.g., a strain of *Candida albicans* or *Aspergillus flavus*. Activity is based on the survival of a treated group of mice after the death of an untreated group of mice. The dose level at which the compound provides 50% protection against the lethal effect of the infection ($PD_{50}$) is noted.

For human use, the antifungal compounds of the formula (I) and their salts, O-ethers and O-esters can be administered alone, but will generally be administered in admixture with a pharmaceutical carrier selected with regard to the intended route of administration and standard pharmaceutical practice. For example, they can be administered orally in the form of tablets containing such excipients as starch or lactose, or in capsules or ovules either alone or in admixture with excipients, or in the form of elixirs or suspensions containing flavouring or colouring agents. They can be injected parenterally, for example, intravenously, intramuscularly or subcutaneously. For parenteral administration, they are best used in the form of a sterile aqueous solution which may contain other substances, for example, enough salts or glucose to make the solution isotonic with blood.

For oral and parenteral administration to human patients, the daily dosage level of the antifungal compounds of the formula (I) and their salts, O-ethers and O-esters will be from 0.1 to 10 mg/kg (in divided doses) when administered by either the oral or parenteral route. Thus tablets or capsules of the compounds will contain from 5 mg to 0.5 g of active compound for administration singly or two or more at a time as appropriate. The physician in any event will determine the actual dosage which will be most suitable for an individual patient and it will vary with the age, weight and response of the particular patient. The above dosages are exemplary of the average case; there can, of course, be individual instances where higher or lower dosage ranges are merited, and such are within the scope of this invention.

Alternatively, the antifungal compounds of formula (I) can be administered in the form of a suppository or pessary, or they may be applied topically in the form of a lotion, solution, cream, ointment or dusting powder. For example, they can be incorporated into a cream consisting of an aqueous emulsion of polyethylene glycols or liquid paraffin; or they can be incorporated, at a concentration between 1 and 10%, into an ointment consisting of a white wax or white soft paraffin base together with such stabilizers and preservatives as may be required.

The compounds of the formula (I) and their O-ethers, O-esters and salts also have activity against a variety of plant pathogenic fungi, including for example various rusts, mildews and moulds, and the compounds are thus useful for treating plants and seeds to eradicate or prevent such diseases.

The *in vitro* evaluation of the activity of the compounds against plant fungi can be determined by measuring their minimum inhibitory concentrations in the same way as previously described except that the plates are incubated at 30°C for 48 hours or longer before being examined for the presence or absence of growth.

Micro-organisms used in such tests include *Cochliobolus carbonum, Pyricularia oryzae, Glomerella cingulata, Penicillium digitatum, Botrytis cinerea* and *Rhizoctonia solani.*

For agricultural and horticultural purposes the compounds and their agriculturally acceptable salts are preferably used in the form of a composition formulated as appropriate to the particular use and purpose desired. Thus the compounds may be applied in the form of dusting powders, or granules, seed dressings, aqueous solutions, dispersions or emulsions, dips, sprays, aerosols or smokes. Compositions may also be supplied in the form of dispersible powders, granules or grains, or concentrates for dilution prior to use. Such compositions may contain such conventional carriers, diluents or adjuvants as are known and acceptable in agriculture and horticulture and they are manufactured in accordance with conventional procedures. The compositions typically contain from 0.01 to 95 wt.%, preferably 0.01 to 1 wt.%, of the active ingredient. The compositions may also incorporate other active ingredients, for example, compounds having herbicidal or insecticidal activity or a further fungicide. The compounds and compositions can be applied in a number of ways, for example they can be applied directly to the plant foliage, stems, branches, seeds or roots or to the soil or other growing medium, and they may be used not only to eradicate disease, but also prophylactically to protect the plants or seeds from attack.

For field use, likely application rates of the active ingredient are from 5 to 500 g/10 ares.

The following Examples illustrate the invention. Temperatures are in °C:—

Example 1

2-(2,4-Dichlorophenyl)-1-(tetrazol-2-yl)-3-(1,2,4-triazol-1-yl)propan-2-ol and 2-(2,4-dichlorophenyl)-1-(tetrazol-1-yl)-3-(1,2,4-triazol-1-yl)propan-2-ol

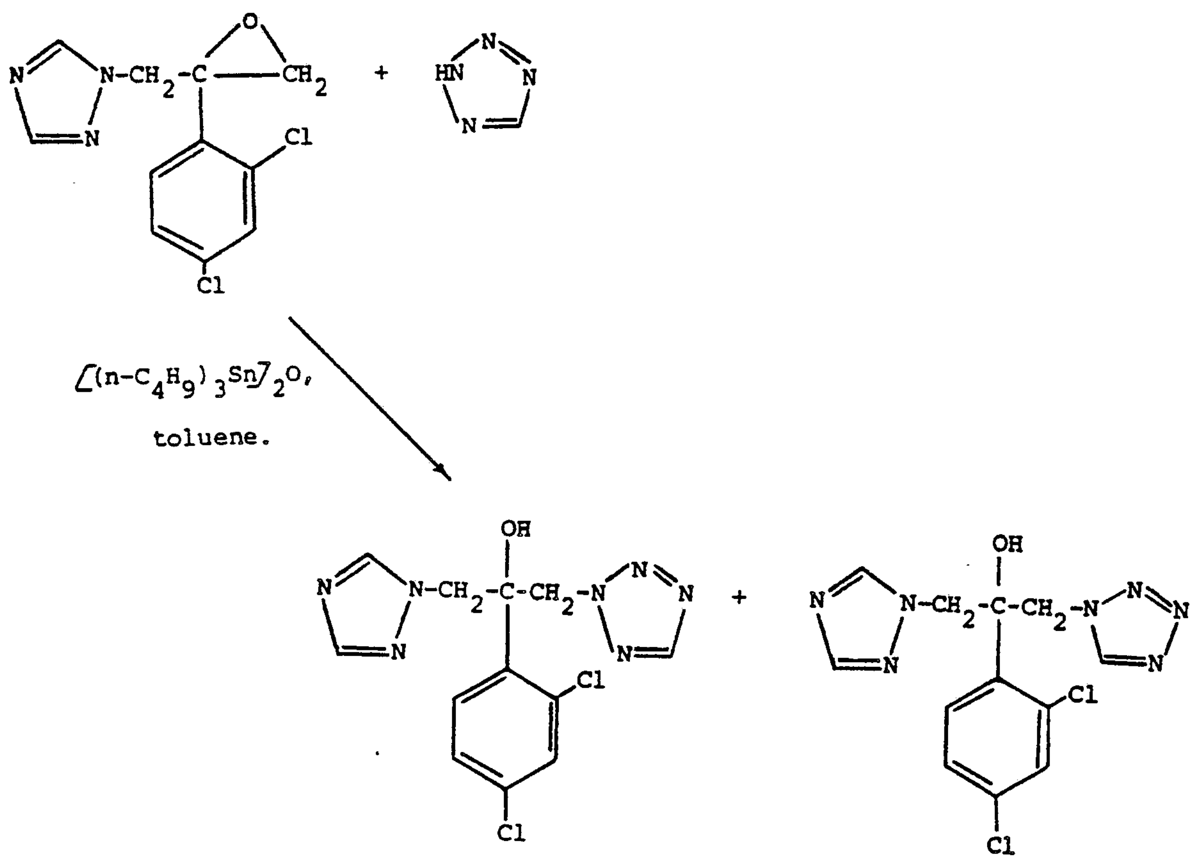


A mixture of 2-(2,4-dichlorophenyl)-2-(1,2,4-triazol-1-ylmethyl)oxirane (2.4 g) [see Example 4 of EP 0044605], tetrazole (750 mg), bis(tri-*n*-butylstannyl)oxide (306 mg) in dry toluene (50 ml) was heated at 100—110° under an atmosphere of nitrogen for 62 hours. Acetic acid (0.5 ml) was then added to the cooled solution, which was filtered and the filtrate after evaporation was dissolved in acetonitrile (150 ml) and then extracted with *n*-hexane (3 × 80 ml). The combined acetonitrile phases were concentrated and the residue was flash chromatographed on silica (230—400 mesh) eluting with a mixture of dichloromethane:methanol:0.88 ammonia (96:4:0.5). The initial fractions gave some unreacted epoxide (424 mg), followed by the tetrazol-2-yl title compound and subsequently the tetrazol-1-yl title compound.

The fractions containing the tetrazol-2-yl isomer were then evaporated, the residue was triturated with hexane and the resulting solid crystallised from ethyl acetate/hexane to yield the title tetrazol-2-yl compound, m.p. 170°, (680 mg). [Mass spec. m/e 340 ($M^+$)].

*Analysis %:—*

| | | | |
|---|---|---|---|
| Found: | C, 42.70; | H, 3.30; | N, 28.48; |
| Calculated for $C_{12}H_{11}Cl_2N_7O$: | C, 42.35; | H, 3.24; | N, 28.82. |

The fractions containing the 1-isomer were evaporated and the residue was recrystallised from ethyl acetate/hexane to yield the title tetrazol-1-yl compound, m.p. 155—156°, (1.086 g). [Mass spec. m/e 340 ($M^+$)].

*Analysis %:—*

| | | | |
|---|---|---|---|
| Found: | C, 43.47; | H, 3.87; | N, 26.07; |
| Calculated for $C_{12}H_{11}Cl_2N_7O$ . 0.4 $CH_3COOC_2H_5$: | C, 43.49; | H, 3.78; | N, 26.11. |

Spectroscopic data confirmed the stated structures.

The $PD_{50}$ values for the above tetrazol-1-yl and tetrazol-2-yl isomers after 48 hours in mice infected with *Candida albicans* are, respectively, 1.1 and 0.32 mg/kg.

Example 2

The following compounds were prepared similarly to Example 1 starting from tetrazole, bis(tri-*n*-butylstannyl)oxide, and 2-(2,4-difluorophenyl)-2-(2-[1,2,4-triazol-1-yl]prop-2-yl) oxirane [see Preparation 1 of EP 0122056] except that the eluant used in the chromatography stage was dichloromethane:methanol:0.88 ammonia in a ratio of 98:2:0.25.

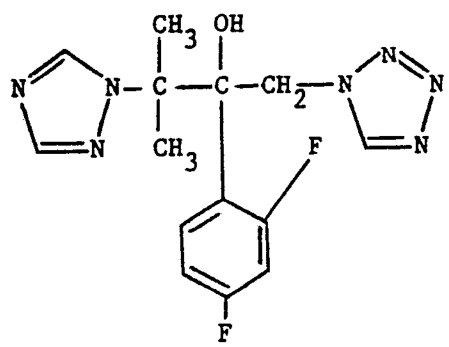

and

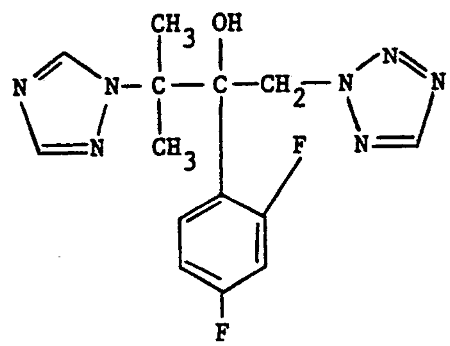

*M.p.* 159—160°

*Analysis %:—*
Found:
C, 50.57; H, 4.69; N, 29.90;
Calculated for $C_{14}H_{15}F_2N_7O$:
C, 50.15; H, 4.48; N, 29.25.

*M.p.* 133—4°

*Analysis %:—*
Found:
C, 49.59; H, 4.64; N, 29.89;
Calculated for $C_{14}H_{15}F_2N_7O$:
C, 50.15; H, 4.48; N, 29.25.

**Claims for the Contracting States: BE CH DE FR GB IT LI LU NL SE**

1. A compound of the formula:—

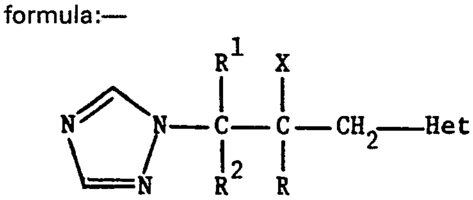

--- (I)

where R is a phenyl group optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group; X is —OH, —F, —Cl or —Br; $R^1$ and $R^2$ are each independently H, $CH_3$ or F; and Het is a 1-tetrazolyl or 2-tetrazolyl group; or an O-ester or O-ether of a compound in which X is —OH;

said O-ester being a $C_2$—$C_4$ alkamoyl or benzoyl ester, said benzoyl group being optionally substituted by 1 or 2 $C_1$—$C_4$ alkyl or halo groups, and said O-ether being a $C_1$—$C_4$ alkyl, $C_2$—$C_4$ alkenyl, phenyl or phenyl-($C_1$—$C_4$ alkyl)ether, said phenyl groups being optionally substituted by 1 or 2 $C_1$—$C_4$ alkyl or halo groups,

or a pharmaceutically or agriculturally acceptable salt of said compounds and esters and ethers.

2. A compound as claimed in claim 1, wherein R is a phenyl group optionally substituted by 1 to 3 substituents each independently selected from F, Cl, Br, I and $CF_3$.

3. A compound as claimed in claim 2, wherein R is a phenyl group substituted by 1 or 2 substituents each selected from F and Cl.

4. A compound as claimed in claim 2, wherein R is 4-fluorophenyl, 4-chlorophenyl, 4-bromophenyl, 4-iodophenyl, 4-trifluoromethylpheny, 2-chlorophenyl, 2,4-dichlorophenyl, 2,4-difluorophenyl, 2-chloro-4-fluorophenyl, 2-fluoro-4-chlorophenyl, 2,5-difluorophenyl, 2,4,6-trifluorophenyl, 4-bromo-2,5-difluorophenyl or 5-chloro-pyrid-2-yl.

5. A compound as claimed in claim 4, wherein R is 2,4-dichlorophenyl or 2,4-difluorophenyl.

6. A compound as claimed in any one of the preceding claims, wherein X is —OH.

7. A compound as claimed in any one of the preceding claims, wherein $R^1$ and $R^2$ are each independently H or $CH_3$.

8. A compound of the formula (I) or an O-ester, O-ether or pharmaceutically acceptable salt thereof as claimed in any one of the preceding claims, for use as a medicament.

9. A pharmaceutical or agricultural fungicidal composition, comprising a compound of the formula (I) or an O-ester, O-ether or pharmaceutically or agriculturally acceptable salt thereof as claimed in any one of claims 1 to 7, and a pharmaceutically or agriculturally acceptable diluent or carrier.

10. A method of treating a plant or seed having a fungal infection, which comprises treating said plant or seed, or the locus thereof, with an antifungally effective amount of a compound of the formula (I) or O-ester, O-ether or agriculturally acceptable salt thereof as claimed in any one of claims 1 to 7.

11. The use of a compound of the formula (I) as claimed in any one of claims 1 to 7, or of a pharmaceutically acceptable salt thereof, for the manufacture of a medicament for use as an antifungal agent.

**Claims for the Contracting State: AT**

1. A process for preparing a compound of the formula:—

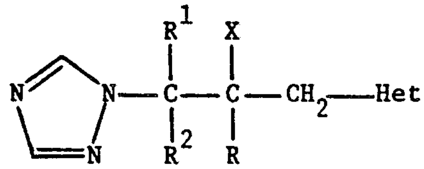

--- (I)

where R is a phenyl group optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group; X is —OH, —F, —Cl or —Br; $R^1$ and $R^2$ are each independently H, $CH_3$ or F; and Het is a 1-tetrazolyl or 2-tetrazolyl group; or an O-ester or O-ether of a compound in which X is —OH;

said O-ester being a $C_2$—$C_4$ alkamoyl or benzoyl ester, said benzoyl group being optionally substituted by 1 or 2 $C_1$—$C_4$ alkyl or halo groups, and said O-ether being a $C_1$—$C_4$ alkyl, $C_2$—$C_4$ alkenyl, phenyl or phenyl-($C_1$—$C_4$ alkyl)ether, said phenyl groups being optionally substituted by 1 or 2 $C_1$—$C_4$ alkyl or halo groups,

or a pharmaceutically or agriculturally acceptable salt of said compounds and esters and ethers, characterised by reacting an oxirane of the formula:—

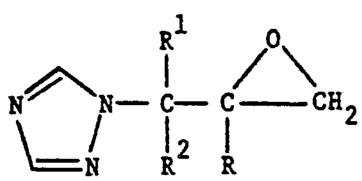

--- (II)

where R, $R^1$ and $R^2$ are as defined above, with tetrazole, thereby producing a compound of the formula (I) in which X is —OH and Het is 1-tetrazolyl in admixture with a compound of the formula (I) in which X is —OH and Het is 2-tetrazolyl; said process being followed by, if desired, one or more of the following steps:—

(a) separating said mixture so as to isolate a compound of the formula (I) in which X is —OH and Het is 1-tetrazolyl, and a compound in which X is —OH and Het is 2-tetrazolyl;

(b) halogenating a compound of the formula (I) in which X is —OH so as to produce a compound of the formula (I) in which X is F, Cl or Br;

(c) converting a compound of the formula (I) in which X is —OH into said O-ester or O-ether; and

(d) converting a compound of the formula (I) or said O-ester or O-ether thereof into a pharmaceutically or agriculturally acceptable salt thereof.

2. A process according to claim 1, characterised in that the reaction of the oxirane (II) with tetrazole is carried out in the presence of bis(tri-*n*-butylstannyl)oxide.

3. A process according to claim 1 or 2, characterised in that it is used to prepare a compound of the formula (I) in which R is a phenyl group substituted by 1 to 3 substituents each independently selected from F, Cl, Br, I and $CF_3$

4. A process according to claim 3, characterised in that it is used to prepare a compound of the formula (I) in which R is a phenyl group substituted by 1 or 2 substituents each selected from F and Cl.

5. A process according to claim 4, characterised in that it is used to prepare a compound of the formula (I) in which R is 2,4-dichlorophenyl or 2,4-difluorophenyl.

6. A process according to any one of the preceding claims, characterised in that it is used to prepare a compound of the formula (I) in which X is —OH and $R^1$ and $R^2$ are each independently H or $CH_3$.

7. A process as claimed in any one of the preceding claims, characterised in that it is carried out in an organic solvent at a temperature of from room temperature to 110°C.

8. A process for preparing a pharmaceutical composition, characterised by mixing a compound of the formula (I), O-ether or O-ester thereof as defined in claim 1, or a pharmaceutically acceptable salt thereof, with a pharmaceutically acceptable diluent or carrier.

9. An agricultural fungicidal composition, comprising a compound of the formula (I), O-ester or O-ether thereof as defined in claim 1, or agriculturally acceptable salt thereof, and an agriculturally acceptable diluent or carrier.

10. A compound of the formula (I), O-ester or O-ether as defined in claim 1, or a pharmaceutically acceptable salt thereof.

**Patentansprüche für die Vertragsstaaten: BE CH DE FR GB IT LI LU NL SE**

1. Verbindung der Formel

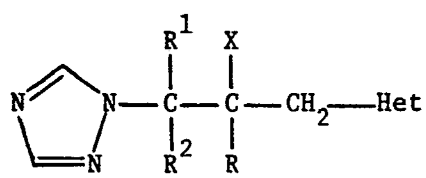

--- (I)

worin R eine Phenylgruppe ist, fakultativ durch 1 bis 3 Substituenten, die jeweils unabhängig voneinander unter F, Cl, Br, J, $CF_3$, $C_1$—$C_4$-Alkyl und $C_1$—$C_4$-Alkoxy ausgewählt sind, substituiert, oder worin R eine 5-Chlorpyrid-2-yl-Gruppe ist, X gleich —OH, —F, —Cl oder —Br ist, $R^1$ und $R^2$ jeweils unabhängig voneinander H, $CH_3$ oder F sind und worin Het eine 1-Tetrazolyl- oder 2-Tetrazolyl-Gruppe ist, oder eines O-Esters oder O-Ethers einer solchen Verbindung, worin X gleich OH ist,

wobei dieser O-Ester ein $C_2$—$C_4$-Alkanoyl- oder Benzoylester ist, die Benzoylgruppe fakultativ durch 1 oder 2 $C_1$—$C_4$-Alkyl oder Halogengruppen substituiert ist und dieser O-Ether ein $C_1$—$C_4$-Alkyl, $C_2$—$C_4$-Alkenyl, Phenyl- oder Phenyl-($C_1$—$C_4$-alkyl)ether ist, wobei die Phenylgruppen fakultativ durch 1 oder 2 $C_1$—$C_4$-Alkyl- oder Halogengruppen substituiert sind;

oder ein pharmazeutisch oder landwirtschaftlich annehmbares Salz dieser Verbindungen und Ester und Ether.

2. Verbindung nach Anspruch 1, worin R eine Phenylgruppe ist, fakultativ substituiert durch 1 bis 3 Substituenten, jeweils unabhängig ausgewählt aus F, Cl, Br, J und $CF_3$.

3. Verbindung nach Anspruch 2, worin R eine Phenylgruppe ist, substituiert durch 1 oder 2 Substituenten, jeweils ausgewählt aus F und Cl.

4. Verbindung nach Anspruch 2, worin R 4-Fluorphenyl, 4-Chlorphenyl, 4-Bromphenyl, 4-Jodphenyl, 4-Trifluormethylphenyl, 2-Chlorphenyl, 2,4-Dichlorphenyl, 2,4-Difluorphenyl, 2-Chlor-4-fluorphenyl, 2-Fluor-4-chlorphenyl, 2,5-Difluorphenyl, 2,4,6-Trifluorphenyl, 4-Brom-2,5-difluorphenyl oder 5-Chlor-pyrid-2-yl ist.

5. Verbindung nach Anspruch 4, worin R 2,4-Dichlorphenyl oder 2,4-Difluorphenyl ist.

6. Verbindung nach irgendeinem der vorhergehenden Ansprüche, worin X —OH ist.

7. Verbindung nach irgendeinem der vorhergehenden Ansprüche, worin $R^1$ und $R^2$ jeweils unabhängig H oder $CH_3$ sind.

8. Verbindung der Formel (I) oder ein O-Ester, O-Ether oder pharmazeutisch annehmbares Salz derselben nach irgendeinem der vorhergehenden Ansprüche zur Verwendung als Medikament.

9. Pharmazeutische oder landwirtschaftliche fungizide Zusammensetzung, umfassend eine Verbindung der Formel (I) oder einen O-Ester, O-Ether oder ein pharmazeutisch oder landwirtschaftlich annehmbares Salz derselben nach irgendeinem der Ansprüche 1 bis 7 und ein pharmazeutisch oder landwirtschaftlich annehmbares Verdünnungsmittel oder einen Träger.

10. Verfahren zum Behandeln einer Pflanze oder von Saatgut mit Pilzinfektion, das umfaßt das Behandeln dieser Pflanze oder dieses Saatgutes oder des Ortes der- bzw. desselben mit einer antifungal wirksamen Menge einer Verbindung der Formel (I) oder eines O-Esters, O-Ethers oder landwirtschaftlich annehmbaren Salzes derselben nach irgendeinem der Ansprüche 1 bis 7.

11. Die Verbindung einer Verbindung der Formel (I) nach irgendeinem der Ansprüche 1 bis 7 oder eines pharmazeutisch annehmbaren Salzes derselben zur Herstellung eines Medikamentes zur Verwendung als antifungales Mittel.

**Patentansprüche für den Vertragsstaat: AT**

1. Verfahren zur Herstellung einer Verbindung mit der Formel

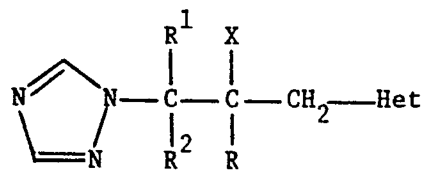

--- (I)

worin R eine Phenylgruppe ist, fakultativ durch 1 bis 3 Substituenten, die jeweils unabhängig voneinander unter F, Cl, Br, J, $CF_3$, $C_1$—$C_4$-Alkyl und $C_1$—$C_4$-Alkoxy ausgewählt sind, substituiert, oder worin R eine 5-Chlorpyrid-2-yl-Gruppe ist, X gleich —OH, —F, —Cl oder —Br ist, $R^1$ und $R^2$ jeweils unabhängig voneinander H, $CH_3$ oder F sind und worin Het eine 1-Tetrazolyl- oder 2-Tetrazolyl-Gruppe ist, oder eines O-Esters oder O-Ethers einer solchen Verbindung, worin X gleich OH ist,

wobei dieser O-Ester ein $C_2$—$C_4$-Alkanoyl- oder Benzoylester ist, die Benzoylgruppe fakultativ durch 1 oder 2 $C_1$—$C_4$-Alkyl- oder Halogengruppen substituiert ist und dieser O-Ether ein $C_1$—$C_4$-Alkyl, $C_2$—$C_4$-Alkenyl-, Phenyl- oder Phenyl-($C_1$—$C_4$-alkyl)ether ist, wobei die Phenylgruppen fakultativ durch 1 oder 2 $C_1$—$C_4$-Alkyl- oder Halogengruppen substituiert sind;

oder eines pharmazeutisch oder landwirtschaftlich annehmbaren Salzes der genannten Verbindungen und Ester und Ether, gekennzeichnet durch Umsetzen eines Oxirans mit der Formel

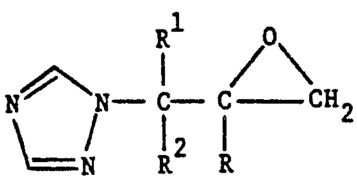

--- (II)

worin R, $R^1$ und $R^2$ wie oben definiert sind, mit Tetrazol, wobei eine Verbindung mit der Formel (I), worin X gleich —OH ist und Het 1-Tetrazolyl ist, im Gemisch mit einer Verbindung mit der Formel (I), worin X gleich —OH ist und Het 2-Tetrazolyl ist, gebildet wird, und wobei diesem Verfahren auf Wunsch einer oder mehrere der folgenden Schritte folgen:

(a) Trennen des Gemischs, um eine Verbindung mit der Formel (I), in welcher X gleich —OH ist und Het 1-Tetrazolyl ist, und eine Verbindung, in welcher X gleich —OH ist und Het 2-Tetrazolyl ist, zu isolieren,

(b) Halogenieren einer Verbindung mit der Formel (I), in welcher X gleich —OH ist, um eine Verbindung mit der Formel (I), in welcher X gleich F, Cl oder Br ist, herzustellen,

(c) Umwandeln einer Verbindung mit der Formel (I), in welcher X gleich —OH ist, in einen O-Ester oder O-Ether, und

(d) Umwandeln einer Verbindung mit der Formel (I) oder eines O-Esters oder O-Ethers hiervon in ein pharmazeutisch oder landwirtschaftlich annehmbares Salz hiervon.

2. Verfahren nach Anspruch 1, dadurch gekennzeichnet, daß die Reaktion des Oxirans (II) mit Tetrazol in Gegenwart von Bis(tri-*n*-butylstannyl)oxid durchgeführt wird.

3. Verfahren nach Anspruch 1 oder 2, dadurch gekennzeichnet, daß es angewendet wird, um eine Verbindung mit der Formel (I) herzustellen, in welcher R eine Phenylgruppe ist, die durch 1 bis 3 Substituenten, die jeweils unabhängig voneinander unter F, Cl, Br, J und $CF_3$ ausgewählt sind, substituiert ist.

4. Verfahren nach Anspruch 3, dadurch gekennzeichnet, daß es angewendet wird, um eine Verbindung mit der Formel (I) herzustellen, in welcher R eine Phenylgruppe ist, die durch 1 oder 2 Substituenten, die jeweils unter F und Cl ausgewählt sind, substituiert ist.

5. Verfahren nach Anspruch 4, dadurch gekennzeichnet, daß es angewendet wird, um eine Verbindung mit der Formel (I) herzustellen, in welcher R 2,4-Dichlorphenyl oder 2,4-Difluorphenyl ist.

6. Verfahren nach einem beliebigen der voranstehenden Ansprüche, dadurch gekennzeichnet, daß es angewendet wird, um eine Verbindung mit der Formel (I) herzustellen, in welcher X gleich —OH ist und $R^1$ und $R^2$ jeweils unabhängig voneinander H oder $CH_3$ sind.

7. Verfahren nach einem beliebigen der voranstehenden Ansprüche, dadurch gekennzeichnet, daß es in einem organischen Solvens bei einer Temperatur von Raumtemperatur bis 110°C durchgeführt wird.

8. Verfahren zur Herstellung einer pharmazeutischen Zusammensetzung, gekennzeichnet durch Vermischen einer Verbindung mit der Formel (I), wie in Anspruch 1 definiert, oder eines O-Ethers, O-Esters

oder pharmazeutisch annehmbaren Salzes hiervon mit einem pharmazeutisch annehmbaren Verdünnungsmittel oder Träger.

9. Landwirtschaftliche, fungizide Zusammensetzung, umfassend eine Verbindung der Formel (I), einen O-Ester oder O-Ether derselben gemäß Definition in Anspruch 1 oder ein landwirtschaftlich annehmbares Salz hiervon und ein landwirtschaftlich annehmbares Verdünnungsmittel oder einen Träger.

10. Verbindung der Formel (I), O-Ester oder O-Ether gemäß Definition in Anspruch 1 oder ein pharmazeutisch annehmbares Salz hiervon.

**Revendications pour les Etats contractants: BE CH DE FR GB IT LI LU NL SE**

1. Composé de formule:

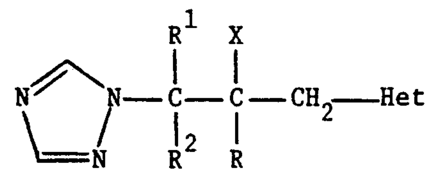

--- (I)

dans laquelle R est un groupe phényle éventuellement substitué par de 1 à 3 substituants, identiques ou différents, choisis parmi F, Cl, Br, I, $CF_3$, alkyle en $C_1$—$C_4$ et alcoxy en $C_1$—$C_4$, ou R est un groupe 5-chloropyrid-2-yle; X représente —OH, —F, —Cl ou —Br; $R^1$ et $R^2$, identiques ou différents, représentent l'hydrogène, $CH_3$ ou F; et Het est un groupe 1-tétrazolyle ou 2-tétrazolyle; ou un O-ester ou O-éther d'un composé dans lequel X représente —OH,

ledit O-ester étant un ester d'alcanoyle en $C_2$—$C_4$ ou de benzoyle, ledit groupe benzoyle étant éventuellement substitué par 1 ou 2 groupes alkyle en $C_1$—$C_4$ ou halogéno, et ledit O-éther étant un éther d'alkyle en $C_1$—$C_4$, d'alkényle en $C_2$—$C_4$, de phényle ou de phényl-(alkyle en $C_1$—$C_4$), lesdits groupes phényle étant éventuellement substitués par 1 ou 2 groupes alkyle en $C_1$—$C_4$ ou halogéno;

ou sel acceptable sur le plan pharmaceutique ou agricole desdits composés, esters et éthers.

2. Composé selon la revendication 1, dans lequel R est un groupe phényle éventuellement substitué par de 1 à 3 substituants, identiques ou différents, choisis entre F, Cl, Br, I et $CF_3$.

3. Composé selon la revendication 2, dans lequel R est un groupe phényle substitué par 1 ou 2 substituants, identiques ou différents, choisis entre F et Cl.

4. Composé selon la revendication 2, dans lequel R est un groupe 4-fluorophényle, 4-chlorophényle, 4-bromophényle, 4-iodophényle, 4-trifluorométhylphényle, 2-chlorophényle, 2,4-dichlorophényle, 2,4-difluorophényle, 2-chloro-4-fluorophényle, 2-fluoro-4-chlorophényle, 2,5-difluorophényle, 2,4,6-trifluorophényle, 4-bromo-2,5-difluorophényle ou 5-chloro-pyrid-2-yle.

5. Composé selon la revendication 4, dans lequel R est un groupe 2,4-dichlorophényle ou 2,4-difluorophényle.

6. Composé selon l'une quelconque des revendications précédentes, dans lequel X représente —OH.

7. Composé selon l'une quelconque des revendications précédentes, dans lequel $R^1$ et $R^2$, identiques ou différents, représentent l'hydrogène ou $CH_3$.

8. Composé de formule (I) ou O-ester, O-éther ou sel pharmaceutiquement acceptable d'un tel composé selon l'une quelconque des revendications précédentes, pour usage en tant que médicament.

9. Composition fongique pharmaceutique ou agricole, comprenant un composé de formule (I) ou un O-ester, un O-éther ou un sel acceptable sur le plan pharmaceutique ou agricole d'un tel composé, selon l'une quelconque des revendications 1 à 7, et un diluant ou véhicule acceptable sur le plan pharmaceutique ou agricole.

10. Méthode pour le traitement d'une plante ou d'une semence ayant une infection fongique, qui consiste à traiter ladite plante ou ladite semence, ou son lieu d'implantation, avec une quantité efficace, sur le plan antifongique, d'un composé de formule (I) ou d'un O-ester, d'un O-éther ou d'un sel acceptable sur le plan agricole d'un tel composé selon l'une quelconque des revendications 1 à 7.

11. Utilisation du composé de formule (I) selon l'une quelconque des revendications 1 à 7, ou d'un sel pharmaceutiquement acceptable d'un tel composé, pour la fabrication d'un médicament employé comme agent antifongique.

**Revendications pour l'Etat contractant: AT**

1. Procédé de préparation d'un composé de formule:

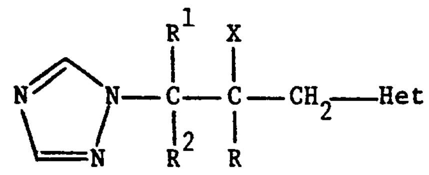

--- (I)

dans laquelle R est un groupe phényle éventuellement substitué par de 1 à 3 substituants, identiques ou différents, choisi parmi F, Cl, Br, I, $CF_3$, alkyle en $C_1$—$C_4$ et alcoxy en $C_1$—$C_4$, ou R est un groupe 5-chloropyrid-2-yle; X représente —OH, —F, —Cl ou —Br; $R^1$ et $R^2$, identiques ou différents, représentent l'hydrogène, $CH_3$ ou F; et Het est un groupe 1-tétrazolyle ou 2-tétrazolyle; ou un O-ester ou O-éther d'un composé dans lequel X représente —OH,

ledit O-ester étant un ester d'alcanoyle en $C_2$—$C_4$ ou de benzoyle, ledit groupe benzoyle étant éventuellement substitué par 1 ou 2 groupes alkyle en $C_1$—$C_4$ ou halogéno, et ledit O-éther étant un éther d'alkyle en $C_1$—$C_4$, d'alkényle en $C_2$—$C_4$, de phényle ou de phényl(alkyle en $C_1$—$C_4$), lesdits groupes phényle étant éventuellement substitués par 1 ou 2 groupes alkyle en $C_1$—$C_4$ ou halogéno;

ou sel acceptable sur le plan pharmaceutique ou agricole de tels composés, esters et éthers, caractérisé en ce que l'on fait réagir un oxirane de formule:

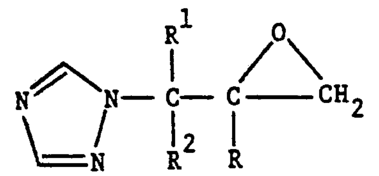
--- (II)

dans laquelle R, $R^1$ et $R^2$ sont tels que définis ci-dessus, avec du tétrazole, de manière à produire un composé de formule (I) dans lequel X représente —OH et Het est un groupe 1-tétrazolyle en mélange avec un composé de formule (I) dans laquelle X représente —OH et Het est un groupe 2-tétrazolyle; ledit procédé étant suivi, si on le désire, d'une ou plusieurs des étapes suivantes:

(a) séparation dudit mélange de façon à isoler un composé de formule (I) dans lequel X représente —OH et Het est un groupe 1-tétrazolyle, et un composé dans lequel X représente —OH et Het est un groupe 2-tétrazolyle;

(b) halogénation d'un composé de formule (I) dans lequel X représente —OH de façon à produire un composé de formule (I) dans lequel X représent F, Cl ou Br;

(c) transformation d'un composé de formule (I) dans lequel X représente —OH en un O-ester ou O-éther, et

(d) transformation d'un composé de formule (I) ou ledit O-ester ou O-éther d'un tel composé, en un sel acceptable sur le plan pharmaceutique ou agricole.

2. Procédé selon la revendication 1, caractérisé en ce que la réaction de l'oxirane (II) avec le tétrazole est effectuée en présence de l'oxyde de bis(tri-*n*-butylstannyle).

3. Procédé selon la revendication 1 ou 2, caractérisé en ce qu'il est utilisé pour préparer un composé de formule (I) dans lequel R est un groupe phényle substitué par de 1 à 3 substituants, identiques ou différents, choisis parmi F, Cl, Br, I et $CF_3$.

4. Procédé selon la revendication 3, caractérisé en ce qu'il est utilisé pour préparer un composé de formule (I) dans lequel R est un groupe phényle substitué par 1 ou 2 substituants choisis parmi F et Cl.

5. Procédé selon la revendication 4, caractérisé en ce qu'il est utilisé pour préparer un composé de formule (I) dans lequel R est un groupe 2,4-dichlorophényle ou 2,4-difluorophényle.

6. Procédé selon l'une quelconque des revendications précédentes, caractérisé en ce qu'il est utilisé pour préparer un composé de formule (I) dans lequel X représente —OH et $R^1$ et $R^2$, identiques ou différents, représentent l'hydrogène ou $CH_3$.

7. Procédé selon l'une quelconque des revendications précédentes, caractérisé en ce qu'il est mis en oeuvre dans un solvant organique à une température comprise entre la température ambiante et 110°C.

8. Procédé de préparation d'une composition pharmaceutique, caractérisée en ce qu'on mélange un composé de formule (I), un O-éther ou O-ester d'un tel composé, tel que défini dans la revendication 1, ou un sel pharmaceutiquement acceptable d'un tel composé, avec un diluant ou véhicule pharmaceutiquement acceptable.

9. Composition fongicide agricole comprenant un composé de formule (I), un O-ester ou O-éther d'un tel composé, tel que défini dans la revendication 1, ou sel acceptable sur le plan agricole d'un tel composé, et un diluant ou véhicule acceptable sur le plan agricole.

10. Composé de formule (I), O-ester ou O-éther tel que défini dans la revendication 1, ou sel pharmaceutiquement acceptable d'un tel composé.